(19) Europäisches Patentamt
European Patent Office
Office européen des brevets

(11) Veröffentlichungsnummer : **0 121 888**
**B1**

(12) **EUROPÄISCHE PATENTSCHRIFT**

(45) Veröffentlichungstag der Patentschrift : **25.05.88**

(21) Anmeldenummer : **84103627.0**

(22) Anmeldetag : **02.04.84**

(51) Int. Cl.⁴ : **C 07 D403/06**, C 07 D231/12, A 01 N 43/64, A 01 N 43/50, A 01 N 43/56// C07D405/06

# (54) 1-Azolyl-3-pyrazolyl-2-propanol-Derivate, Verfahren zu ihrer Herstellung und ihre Verwendung als Fungizide.

(30) Priorität : **12.04.83 DE 3313073**

(43) Veröffentlichungstag der Anmeldung : **17.10.84 Patentblatt 84/42**

(45) Bekanntmachung des Hinweises auf die Patenterteilung : **25.05.88 Patentblatt 88/21**

(84) Benannte Vertragsstaaten : **AT BE CH DE FR GB IT LI NL SE**

(56) Entgegenhaltungen :
**EP-A- 0 044 605**
**EP-A- 0 069 442**
**TETRAHEDRON, Band 39, Nr. 12, 1983, Seiten 2023-2029, Pergamon Press, GB; A.R. KATRITZKY et al.: "Alpha-lithiation of N-alkyl groups in pyrazoles"**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Regel, Erik, Dipl.-Ing.**
**Untere Bergerheide 26**
**D-5600 Wuppertal 1 (DE)**
Erfinder : **Büchel, Karl Heinz, Prof. Dr.**
**Dabringhausener Strasse 42**
**D-5093 Burscheid (DE)**
Erfinder : **Reinecke, Paul, Dr.**
**Lessingstrasse 11**
**D-5090 Leverkusen 3 (DE)**
Erfinder : **Brandes, Wilhelm, Dr.**
**Eichendorffstrasse 3**
**D-5653 Leichlingen 1 (DE)**

**EP 0 121 888 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue 1-Azolyl-3-pyrazolyl-2-propanol-Derivate, mehrere Verfahren zu ihrer Herstellung sowie ihre Verwendung als Fungizide.

Es ist bereits bekannt geworden, daß bestimmte, 1,3-Diazolyl-2-propanol-Derivate, wie beispielsweise 1,3-Di(1,2,4-triazol-1-yl)-2-(4-chlorphenyl)-2-propanol, gute fungizide Eigenschaften aufweisen (vergleiche EP-A 0 044 605). Die Wirkung dieser Verbindungen ist jedoch, insbesondere bei niedrigen Aufwandmengen und -konzentrationen, nicht immer voll befriedigend.

Es wurden neue 1-Azolyl-3-pyrazolyl-2-propanol-Derivate der allgemeinen Formel

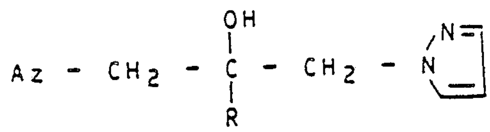

(I)

in welcher

Az für 1,2,4-Triazol-1-yl, Imidazol-1-yl oder Pyrazol-1-yl steht und

R für geradkettiges oder verzweigtes Alkyl mit 1 bis 12 Kohlenstoffatomen, geradkettiges oder verzweigtes Alkenyl und Alkinyl mit jeweils 2 bis 6 Kohlenstoffatomen steht, für jeweils im Phenylteil gegebenenfalls einfach bis dreifach, gleich oder verschieden durch Halogen, Alkyl mit 1 bis 4 Kohlenstoffatomen, Alkoxy und Alkylthio mit jeweils 1 oder 2 Kohlenstoffatomen, Alkoxyalkyl mit 1 oder 2 Kohlenstoffatomen in jedem Alkylteil sowie durch gegebenenfalls halogensubstituiertes Phenyl, substituiertes Phenyl, Phenylalkyl mit 1 bis 4 Kohlenstoffatomen im Alkylteil, Phenylalkenyl mit 2 bis 4 Kohlenstoffatomen im Alkenylteil und Phenylalkinyl mit 2 bis 4 Kohlenstoffatomen im Alkinylteil steht, ferner für Naphthyl sowie gegebenenfalls durch Alkyl mit 1 bis 4 Kohlenstoffatomen substituiertes Cycloalkyl mit 3 bis 7 Kohlenstoffatomen steht,

sowie deren Säureadditions-Salze und Metallsalz-Komplexe gefunden.

Weiterhin wurde gefunden, daß man die 1-Azolyl-3-pyrazolyl-2-propanol-Derivate der Formel (I) sowie deren Säureadditions-Salze und Metallsalz-Komplexe erhält, wenn man

a) 2-Azolylmethyl-oxirane der Formel

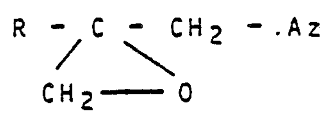

(II)

in welcher Az und R die oben angegebene Bedeutung haben, mit Azolen der Formel

H—Az' (III)

in welcher Az' für die Bedeutungen von Az steht, wobei jedoch Az oder/und Az' für Pyrazol-1-yl stehen, in Gegenwart eines Alkalialkoholats und in Gegenwart eines Verdünnungsmittels umsetzt, oder

b) 2-Halogenmethyl-oxirane der Formel

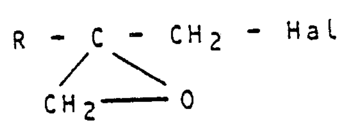

(IV)

in welcher

R die oben angegebene Bedeutung hat und

Hal für Halogen steht,

mit Pyrazol der Formel

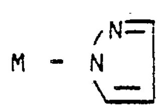

(V)

in welcher M für Wasserstoff oder ein Alkalimetall steht, in Gegenwart eines Verdünnungsmittels und

gegebenenfalls in Gegenwart eines Säurebindemittels umsetzt, oder

c) Dihalogenalkanole der Formel

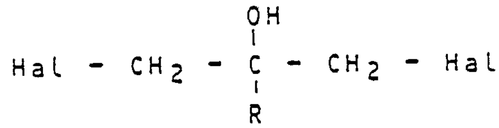

(VI)

in welcher Hal und R die oben angegebene Bedeutung haben, mit Pyrazol der Formel (V) in Gegenwart eines Verdünnungsmittels und gegebenenfalls in Gegenwart eines Säurebindemittels umsetzt, und gegebenenfalls an die so erhaltenen Verbindungen der Formel (I) anschließend eine Säure oder ein Metallsalz addiert.

Ueberraschenderweise zeigen die erfindungsgemäßen Verbindungen der Formel (I) eine bessere fungizide Wirksamkeit als das aus dem Stand der Technik bekannte 1,3-Di-(1,2,4-triazol-1-yl)-2-(4-chlorphenyl)-2-propanol, welches konstitutionell und wirkungsmäßig eine naheliegende Verbindung ist. Die erfindungsgemäßen Wirkstoffe stellen somit eine Bereicherung der Technik dar.

Die erfindungsgemäßen 1-Azolyl-3-pyrazolyl-2-propanol-Derivate sind durch die Formel (I) allgemein definiert.

Bevorzugt sind diejenigen Verbindungen der Formel (I), in denen

Az für 1,2,4-Triazol-1-yl, Imidazol-1-yl oder Pyrazol-1-yl steht und

R für Ethyl, tert.-Butyl, Allyl oder Propargyl steht, ferner für jeweils gegebenenfalls im Phenylteil einfach bis zweifach, gleich oder verschieden substituiertes Phenyl, Benzyl, Phenethyl, Phenethenyl und Phenethinyl steht, wobei als Phenylsubstituenten jeweils genannt seien : Fluor, Chlor, Methyl, Methoxy, Methylthio, Isopropoxy, Methoxymethyl und gegebenenfalls durch Fluor oder Chlor substituiertes Phenyl, sowie für Naphthyl und jeweils gegebenenfalls durch Methyl oder Ethyl substituiertes Cyclopropyl, Cyclopentyl oder Cyclohexyl steht.

Besonders bevorzugt sind diejenigen Verbindungen der Formel (I), in denen

Az für 1,2,4-Triazol-1-yl, Imidazol-1-yl oder Pyrazol-1-yl steht und

R für gegebenenfalls einfach oder zweifach, gleich oder verschieden substituiertes Phenyl steht, wobei als Substituenten genannt seien : Fluor, Chlor, Methyl und Methoxy.

Bevorzugte erfindungsgemäße Verbindungen sind auch Additionsprodukte aus Säuren und denjenigen 1-Azolyl-3-pyrazolyl-2-propanol-Derivaten der Formel (I), in denen die Substituenten Az und R die Bedeutungen haben, die bereits als bevorzugt für diese Substituenten genannt wurden.

Zu den Säuren, die addiert werden können, gehören vorzugsweise Halogenwasserstoffsäuren, wie z. B. Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsaüren, wie z. B. Essigsäure, Maleinsäure, Bernsteinsäure, Fumarsäure, Weinsäure, Zitronensäure, Salizylsäure, Sorbinsäure und Milchsäure, sowie Sulfonsaüren, wie p-Toluolsulfonsäure und 1,5-Naphthalindisulfonsäure.

Außerdem bevorzugte erfindungsgemäße Verbindungen sind Additionsprodukte aus Salzen und Metallen der II. bis IV. Haupt- und der I. und II. sowie IV. bis VIII. Nebengruppe und denjenigen 1-Azolyl-3-pyrazolyl-2-propanol-Derivaten der Formel (I), in denen die Substituenten Az und R die Bedeutungen haben, die bereits als bevorzugt für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z. B. die Chlorwasserstoffsäure und die Bromwasserstoffsäure, Salpetersäure und Schwefelsäure.

Verwendet man beispielsweise 2-(2-Chlorphenyl)-2-(1,2,4-triazol-1-yl-methyl)-oxiran, Kalium-tert.-butylat und Pyrazol als Ausgangsstoffe, so kann der Reaktionsablauf des erfindungsgemäßen Verfahrens (a) durch das folgende Formelschema wiedergegeben werden :

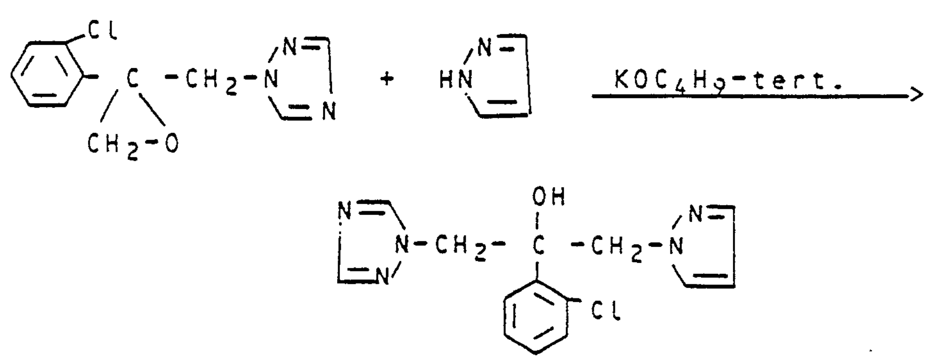

Verwendet man beispielsweise 2 Chlormethyl-2-(4-chlorphenyl)-oxiran und Pyrazol als Ausgangsstoffe sowie Kaliumcarbonat als Säurebindemittel, so kann der Reaktionsablauf des erfindungsgemäßen Verfahrens (b) durch das folgende Formelschema wiedergegeben werden :

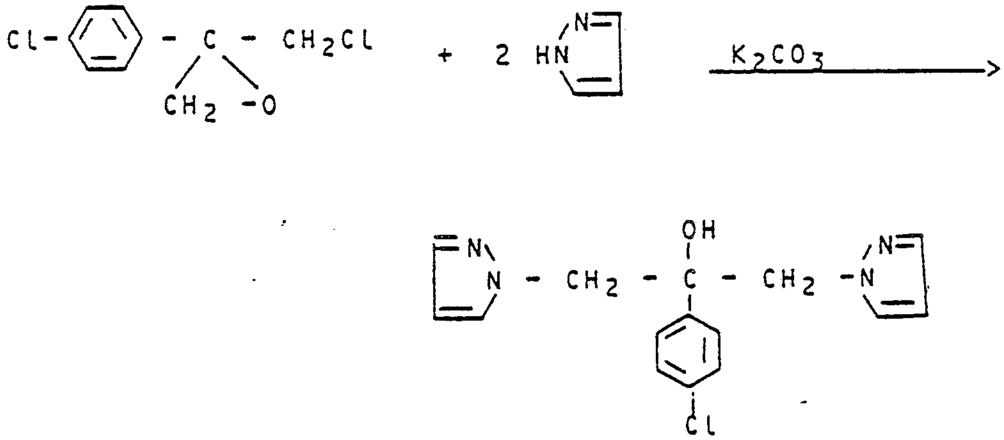


Verwendet man beispielsweise 1,3-Dichlor-2-(4-chlorphenyl)-2-propanol und Pyrazol als Ausgangsstoffe sowie Kaliumcarbonat als Säurebindemittel, so kann der Ablauf des erfindungsgemäßen Verfahrens (c) durch das folgende Formelschema wiedergegeben werden :

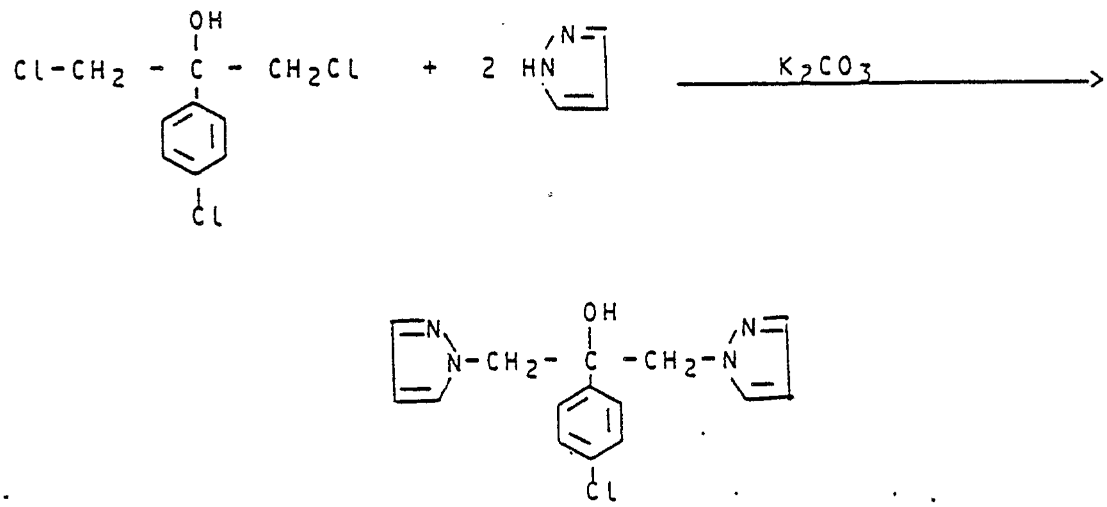


Die für das erfindungsgemäße Verfahren (a) als Ausgangsstoffe zu verwendenden 2-Azolylmethyl-oxirane sind durch die Formel (II) allgemein definiert. In dieser Formel stehen Az und R vorzugsweise für diejenigen Reste, die bereits im Zusammenhang mit der Beschreibung der erfindungsgemäßen Stoffe der Formel (I) als bevorzugt für diese Substituenten genannt wurden.

Die Azolylmethyl-oxirane der Formel (II) sind teilweise bekannt (vergleiche z. B. EP-A 0 044 605 und EP-A 0 061 835). Noch nicht bekannt sind die 2-Pyrazolylmethyl-oxirane der Formel

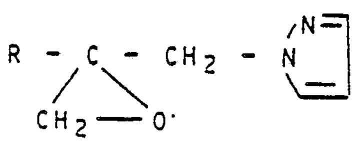 (IIa)

in welcher R die oben angegebene Bedeutung hat.

Die 2-Azolylmethyl-oxirane der Formel (II) können erhalten werden, indem man 2-Halogenmethyloxirane der Formel (IV) mit Azolen der Formel (III) in Gegenwart eines inerten organischen Lösungsmittels, wie beispielsweise Aceton, und in Gegenwart eines Säurebindemittels, wie beispielsweise Kaliumcarbonat, bei Temperaturen zwischen 20 und 120 °C umsetzt (vergleiche auch die Herstellungsbeispiele).

Die 2-Azolylmethyl-oxirane der Formel (II) können in allgemein bekannter Art und Weise auch erhalten werden, indem man Azolo-ketone der Formel

$$R—CO—CH_2—Az \quad (VII)$$

in welcher Az und R die oben angegebene Bedeutung haben, entweder

α) mit Dimethyloxosulfonium-methylid der Formel

$$\overset{\delta^+\ \delta^-}{(CH_3)_2SOCH_2} \quad (VIII)$$

in an sich bekannter Weise in Gegenwart eines Verdünnungsmittels, wie beispielsweise Dimethylsulfoxid, bei Temperaturen zwischen 20 und 80 °C umsetzt (vergleiche hierzu die Angaben in J. Am. Chem. Soc. 87, 1363-1364 (1965)), oder

β) mit Trimethylsulfonium-methylsulfat der Formel

$$[(CH_3)_3S^{(+)}]\ CH_3SO_4^{(-)} \quad (IX)$$

in an sich bekannter Weise in Gegenwart eines inerten organischen Lösungsmittels, wie z. B. Acetonitril, und in Gegegenwart einer Base, wie z. B. Natriummethylat, bei Temperaturen zwischen 0 bis 60 °C, vorzugsweise bei Raumtemperatur, umsetzt (vergleiche auch die Angaben in Heterocycles 8, 397 (1977)).

Die so erhaltenen Oxirane der Formel (II) können gegebenenfalls ohne Isolierung direkt weiter umgesetzt werden.

Die bei der Herstellung der 2-Azolylmethyl-oxirane der Formel (II) als Ausgangsstoffe benötigten Ketone der Formel (VII) sind bekannt (vergleiche beispielsweise DE-A 24 31 407, DE-A 26 38 470, DE-A 28 20 361 bzw, DE-A 30 48 266), bzw. lassen sich nach im Prinzip bekannten Verfahren herstellen.

Das bei der Verfahrensvariante (α) benötigte Dimethyloxosulfoniummethylid der Formel (VIII) ist ebenfalls bekannt (vergleiche J. Am. Chem. Soc. 87, 1363-1364 (1965)). Es wird bei der obigen Umsetzung in frisch zubereitetem Zustand verarbeitet, indem man es in situ durch Umsetzung von Trimethyloxosulfoniumiodid mit Natriumhydrid oder Natriumamid, insbesondere mit Kalium-tert.-butylat oder Natriummethylat, in Gegenwart eines Verdünnungsmittels erzeugt.

Das bei der Verfahrensvariante (β) benötigte Trimethylsulfoniummethylsulfat der Formel (IX) ist ebenfalls bekannt (vergleiche Heterocycles 8, 397 (1977)). Es wird bei der obigen Umsetzung ebenfalls in frisch hergestelltem Zustand eingesetzt, indem man es durch Reaktion von Dimethylsulfid mit Dimethylsulfat in situ erzeugt.

Die Azole der Formel (III) sind allgemein bekannte Verbindungen der organischen Chemie.

Die für das erfindungsgemäße Verfahren (b) als Ausgangsstoffe zu verwendenden 2-Halogenmethyloxirane sind durch die Formel (IV) allgemein definiert. In dieser Formel steht R vorzugsweise für diejenigen Reste, die bereits im Zusammenhang mit der Beschreibung der erfindungsgemäßen Stoffe der Formel (I) als bevorzugt für diesen Substituenten genannt wurden. Hal steht vorzugsweise für Chlor, Brom oder Iod.

Die 2-Halogenmethyl-oxirane der Formel (IV) sind bekannt, bzw. können sie in allgemein bekannter Art und Weise erhalten werden, indem man Dihalogenalkanole der Formel (VI) mit wässriger Alkalilauge versetzt (vergleiche hierzu auch J. Org. Chem. 27, 2242 (1961)).

Die außerdem für das erfindungsgemäße Verfahren (b) als Ausgangsstoffe zu verwendenden Azole sind durch die Formel (V) allgemein definiert. In dieser Formel steht M vorzugsweise für Wasserstoff, Natrium oder Kalium.

Die Azole der Formel (V) sind allgemein bekannte Verbindungen der organischen Chemie. Die Alkalisalze werden durch Umsetzung von Pyrazol, Imidazol bzw. 1,2,4-Triazol mit Natrium- oder Kaliumethylat oder durch Umsetzung von Pyrazol, Imidazol bzw. 1,2,4-Triazol mit der äquivalenten Mengen des entsprechenden Alkalihydrids erhalten.

Die für das erfindungsgemäße Verfahren (c) als Ausgangsstoffe zu verwendenden Dihalogenalkanole sind durch die Formel (VI) allgemein definiert. In dieser Formel steht R vorzugsweise für diejenigen Reste, die bereits im Zusammenhang mit der Beschreibung der erfindungsgemäßen Stoffe der Formel (I) als bevorzugt für diesen Substituenten genannt wurden. Hal steht vorzugsweise für Chlor.

Die Dihalogenalkanole der Formel (VI) sind bekannt bzw. können sie in allgemein bekannter Art und Weise erhalten werden, indem man 1,3-Dihalogenacetone, wie insbesondere 1,3-Dichloraceton, mit einem entsprechenden Grignardreagenz umsetzt (vergleiche hierzu auch J. Org. Chem. 27, 2242 (1961)).

Als Verdünnungsmittel kommen für das erfindungsgemäße Verfahren (a) vorzugsweise inerte organische Lösungsmittel infrage. Hierzu gehören vorzugsweise Nitrile, wie insbesondere Acetonitril ; aromatische Kohlenwasserstoffe, wie Benzol, Toluol und Dichlorbenzol ; Formamide, wie insbesondere Dimethylformamid ; halogenierte Kohlenwasserstoffe, wie Methylenchlorid oder Tetrachlorkohlenstoff ; sowie Hexamethylphosphorsäuretriamid.

Das erfindungsgemäße Verfahren (a) wird in Gegenwart eines Alkalialkoholats durchgeführt. Hierzu gehören vorzugsweise die Methylate und Ethylate von Natrium und Kalium sowie insbesondere auch Kalium-tert.-butylat.

Die Reaktionstemperaturen können bei der Durchführung des erfindungsgemäßen Verfahrens (a) in einem größeren Bereich variiert werden. Im allgemeinen arbeitet man zwischen etwa 30 bis 150 °C, vorzugsweise zwischen 80 und 100 °C.

Bei der Durchführung des erfindungsgemäßen Verfahrens (a) setzt man auf 1 Mol Oxiran der Formel (II) vorzugsweise 1 bis 4 Mol Azol der Formel (III) und 1 bis 4 Mol Alkalialkoholat ein. Die Isolierung der Endprodukte erfolgt in allgemein üblicher Weise.

In einer bevorzugten Ausführungsform des erfindungsgemäßen Verfahrens (a) werden die gemäß Verfahren (α) oder (β) erhaltenen Oxirane der Formel (II) ohne Isolierung direkt weiter umgesetzt.

Als Verdünnungsmittel kommen für die erfindungsgemäßen Verfahren (b) und (c) vorzugsweise inerte organische Lösungsmittel infrage. Hierzu gehören vorzugsweise Ketone, wie insbesondere Aceton und Methylethylketon ; Nitrile, wie insbesondere Acetonitril ; Alkohole wie insbesondere Ethanol und Isopropanol ; Ether, wie insbesondere Tetrahydrofuran oder Dioxan ; Formamide, wie insbesondere Dimethylformamid ; sowie aromatische und halogenierte Kohlenwasserstoffe.

Die erfindungsgemäßen Verfahren (b) und (c) werden gegebenenfalls in Gegenwart eines Säurebindemittels vorgenommen. Man kann alle üblicherweise verwendbaren anorganischen oder organischen Säurebinder zugeben, wie Alkalicarbonate, beispielsweise Natrium- und Kaliumcarbonat ; oder wie niedere tertiäre Alkylamine, Cycloalkylamine oder Aralkylamine, beispielsweise Triethylamin, N,N-Dimethylcyclohexylamin, Dicyclohexylamin, N,N-Dimethylbenzylamin, weiterhin Pyridin und Diazabicyclooctan sowie auch einen entsprechenden Überschuß an Azol.

Die Reaktionstemperaturen können bei der Durchführung der erfindungsgemäßen Verfahren (b) und (c) in einem größeren Bereich variiert werden. Im allgemeinen arbeitet man zwischen etwa 20 bis etwa 150 °C, vorzugsweise bei 20 bis 120 °C.

Bei der Durchführung der erfindungsgemäßen Verfahren (b) und (c) setzt man auf 1 Mol der 2-Halogenmethyloxirane der Formel (IV) bzw. der Dihalogenalkanole der Formel (VI) 2 bis 4 Mol Azol der Formel (V) und gegebenenfalls 2 bis 4 Mol Säurebinder ein. Die Isolierung der Verbindungen der Formel (I) erfolgt in üblicher Art und Weise.

Die Säureadditionssalze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Die Metallsalz-Komplexe der Verbindungen der Formel (I) können in einfacher Weise nach üblichen Verfahren erhalten werden, so z. B. durch Lösen des Metallsalzes in Alkohol, z. B. Ethanol und Hinzufügen zu Verbindungen der Formel (I). Man kann Metallsalz-Komplexe in bekannter Weise, z. B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zu Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Getreidekrankheiten, wie Cochliobolus sativus, Erysiphe graminis, Puccinia und Pyrenophora teres ; ferner zur Bekämpfung des Apfelschorfs (Venturia inaequalis) und von Reiskrankheiten, wie Pyricularia und Pellicularia, eingesetzt werden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff

und Kohlendioxid ; als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z. B. nichtionogene und anionische Emulgatoren, wie Polyoxyethylen-Fettsäure-Ester, Polyoxyethylen-Fettalkohol-Ether, z. B. Alkylarylpolyglykol-Ether, Alkylsulfonate, Alkylsulfate, Arylsulfonate sowie Eiweißhydrolysate ; als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise, 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

## Herstellungsbeispiele

### Beispiel 1

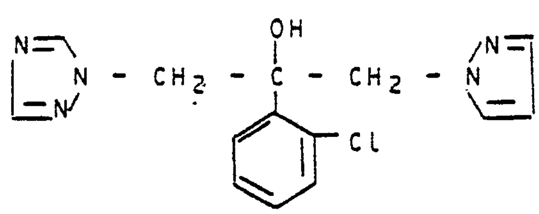

(Verfahren a)

Ein Gemisch von 3,4 g (50 mMol) Pyrazol, 2,8 g (25 mMol) Kalium-tert.-butylat und 5,9 g (25 mMol) 2-(2-Chlorphenyl)-2-(1,2,4-triazol1-yl-methyl)-oxiran in 200 ml Dimethylformamid wird 36 Stunden auf 100 °C erhitzt. Das Reaktionsgemisch wird anschließend im Vakuum eingeengt, der Rückstand in Chloroform gelöst, filtriert, und mit Wasser gewaschen. Die Chloroformlösung wird über Natriumsulfat getrocknet, filtriert und im Vakuum eingeengt. Der verbleibende Rückstand wird chromatographisch gereinigt (Kieselgel 60-Merck/Chloroform). Man erhält 5,8 g (74 % der Theorie) 2-(2-Chlorphenyl)-1-(pyrazol-1-yl)-3-(1,2,4-triazol-1-yl)-2-propanol vom Brechungsindex $n^{20}_D = 1{,}5690$.

Herstellung des Ausgangsproduktes

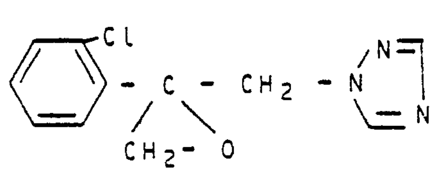

Eine Lösung von 68,5 g (0,33 Mol) 2-(2-Chlorphenyl)-2-chlormethyl-oxiran in 50 ml Aceton wird eingetropft in ein Gemisch von 24,2 g (0,35 Mol) Triazol und 48,3 g (0,35 Mol) Kaliumcarbonat in 300 ml Aceton. Das Reaktionsgemisch wird 20 Stunden unter Rückfluß erhitzt, filtriert und das Filtrat eingeengt. Der Rückstand wird in Methylenchlorid gelöst, mit Wasser gewaschen, über Natriumsulfat getrocknet, filtriert und erneut eingeengt. Der Rückstand wird säulenchromatographisch gereinigt (Kieselgel 60-Merck/Chloroform). Man erhält 159 g (20,5 % der Theorie) 2-(2-Chlorphenyl)-2-(1,2,4-triazol-1-yl-methyl)-oxiran vom Brechungsindex $n^{20}_D = 1{,}5572$.

In entsprechender Weise und gemäß den angegebenen Verfahren werden die nachfolgenden Verbindungen der allgemeinen Formel

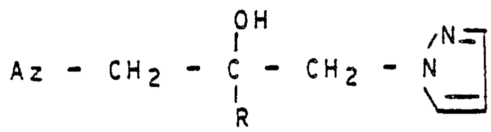

erhalten :

| Bsp.Nr. | R | Az | Schmelzpunkt (°C) |
|---|---|---|---|
| 2 | 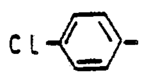 | 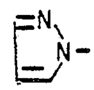 | 118 |
| 3 | 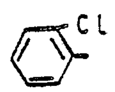 | 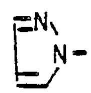 | 100 |

## Anwendungsbeispiele

In den nachfolgenden Anwendungsbeispielen wird die nachstehend angegebene Verbindung als Vergleichssubstanz eingesetzt :

(A) 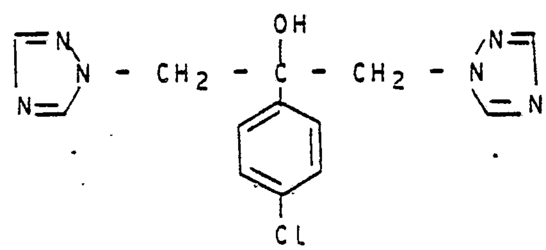

### Beispiel A

Cochliobolus sativus-Test (Gerste)/protektiv

Lösungsmittel : 100 Gewichtsteile Dimethylformamid
Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20 °C und 100 % rel. Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

Eine deutliche Ueberlegenheit in der Wirksamkeit gegenüber dem Stand der Technik zeigen bei diesem Test z. B. die Verbindungen gemäß folgender Herstellungsbeispiele : 1

Beispiel B

Venturia-Test (Apfel)/protektiv

Lösungsmittel : 4,7 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele : 1.

**Patentansprüche**

1. 1-Azolyl-3-pyrazolyl-2-propanol-Derivate der allgemeinen Formel

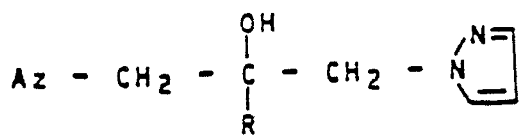

(I)

in welcher

Az für 1,2,4-Triazol-1yl, Imidazol-1-yl oder Pyrazol-1-yl steht und

R für geradkettiges oder verzweigtes Alkyl mit 1 bis 12 Kohlenstoffatomen, geradkettiges oder verzweigtes Alkenyl und Alkinyl mit jeweils 2 bis 6 Kohlenstoffatomen steht, für jeweils im Phenylteil gegebenenfalls einfach bis dreifach, gleich oder verschieden durch Halogen, Alkyl mit 1 bis 4 Kohlenstoffatomen, Alkoxy und Alkylthio mit jeweils 1 oder 2 Kohlenstoffatomen, Alkoxyalkyl mit 1 oder 2 Kohlenstoffatomen in jedem Alkylteil sowie durch gegebenenfalls halogensubstituiertes Phenyl, substituiertes Phenyl, Phenylalkyl mit 1 bis 4 Kohlenstoffatomen im Alkylteil, Phenylalkenyl mit 2 bis 4 Kohlenstoffatomen im Alkenylteil und Phenylalkinyl mit 2 bis 4 Kohlenstoffatomen im Alkinylteil steht, ferner für Naphthyl sowie gegebenenfalls durch Alkyl mit 1 bis 4 Kohlenstoffatomen substituiertes Cycloalkyl mit 3 bis 7 Kohlenstoffatomen steht,

sowie deren Säureadditions-Salze und Metallsalz-Komplexe.

2. Verbindungen der Formel (I) in Anspruch 1, wobei

Az für 1,2,4-Triazol-1-yl, Imidazol-1-yl oder Pyrazol-1-yl steht und

R für gegebenenfalls einfach oder zweifach, gleich oder verschieden durch Fluor, Chlor, Methyl und Methoxy substituiertes Phenyl steht.

3. Verfahren zur Herstellung von 1-Azolyl-3-pyrazolyl-2-propanol-Derivaten der allgemeinen Formel

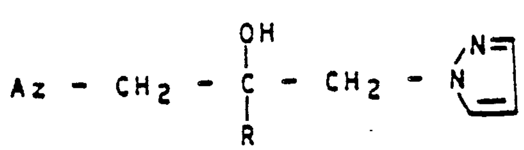

(I)

in welcher

Az für 1,2,4-Triazol-1-yl, Imidazol-1-yl oder Pyrazol-1-yl steht und

R für geradkettiges oder verzweigtes Alkyl mit 1 bis 12 Kohlenstoffatomen, geradkettiges oder verzweigtes Alkenyl und Alkinyl mit jeweils 2 bis 6 Kohlenstoffatomen steht, für jeweils im Phenylteil gegebenenfalls einfach bis dreifach, gleich oder verschieden durch Halogen, Alkyl mit 1 bis 4 Kohlenstoffatomen, Alkoxy und Alkylthio mit jeweils 1 oder 2 Kohlenstoffatomen, Alkoxyalkyl mit 1 oder 2 Kohlenstoffatomen in jedem Alkylteil sowie durch gegebenenfalls halogensubstituiertes Phenyl, substituiertes Phenyl, Phenylalkyl mit 1 bis 4 Kohlenstoffatomen im Alkylteil, Phenylalkenyl mit 2 bis 4 Kohlenstoffatomen im Alkenylteil und Phenylalkinyl mit 2 bis 4 Kohlenstoffatomen im Alkinylteil steht, ferner für Naphthyl sowie gegebenenfalls durch Alkyl mit 1 bis 4 Kohlenstoffatomen substituiertes

Cycloalkyl mit 3 bis 7 Kohlenstoffatomen steht,
sowie von deren Säureadditions-Salzen und Metallsalz-Komplexen,
dadurch gekennzeichnet, daß man
a) 2-Azolylmethyl-oxirane der Formel

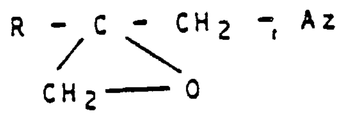

(II)

in welcher Az und R die oben angegebene Bedeutung haben, mit Azolen der Formel

H—Az' (III)

in welcher Az' für die Bedeutungen von Az steht, wobei jedoch Az und/oder Az' für Pyrazol-1-yl stehen, in Gegenwart eines Alkalialkoholats und in Gegenwart eines Verdünnungsmittels umsetzt, oder
b) 2-Halogenmethyl-oxirane der Formel

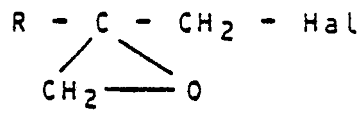

(IV)

in welcher
R die oben angegebene Bedeutung hat und
Hal für Halogen steht,
mit Pyrazol der Formel

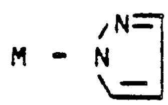

(V)

in welcher M für Wasserstoff oder ein Alkalimetall steht, in Gegenwart eines Verdünnungsmittels und gegebenenfalls in Gegenwart eines Säurebindemittels umsetzt, oder
c) Dihalogenalkanole der Formel

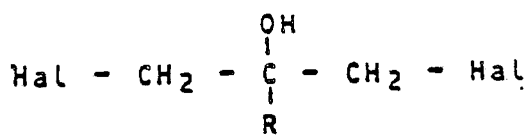

(VI)

in welcher Hal und R die oben angegebene Bedeutung haben, mit Pyrazol der Formel (V) in Gegenwart eines Verdünnungsmittels und gegebenenfalls in Gegenwart eines Säurebindemittels umsetzt,
und gegebenenfalls an die so erhaltenen Verbindungen der Formel (I) anschließend eine Säure oder ein Metallsalz addiert.

4. Fungizide Mittel, gekennzeichnet durch einen Gehalt an mindestens einem 1-Azolyl-2-pyrazolyl-2-propanol-Derivat der Formel (I) gemäß Anspruch 1 oder einem Säureadditions-Salz oder Metallsalz-Komplex eines 1-Azolyl-3-pyrazolyl-2-propanol-Derivates der Formel (I).

5. Verfahren zur Bekämpfung von Pilzen, dadurch gekennzeichnet, daß man 1-Azolyl-3-pyrazolyl-2-propanol-Derivate der Formel (I) in Anspruch 1 bzw. deren Säureadditions-Salze oder Metallsalz-Komplexe auf Pilze oder ihren Lebensraum einwirken läßt.

6. Verwendung von 1-Azolyl-3-pyrazolyl-2-propanol-Derivaten der Formel (I) in Anspruch 1 bzw. von deren Säureadditions-Salzen oder Metallsalz-Komplexen zur Bekämpfung von Pilzen.

7. Verfahren zur Herstellung von fungiziden Mitteln, dadurch gekennzeichnet, daß man 1-Azolyl-3-pyrazolyl-2-propanol-Derivate der Formel (I) in Anspruch 1 bzw. deren Säureadditions-Salze oder Metallsalz-Komplexe mit Streckmitteln und/oder oberflächenaktiven Mitteln vermischt.

**Claims**

1. 1-Azolyl-3-pyrazolyl-2-propanol derivatives of the general formula

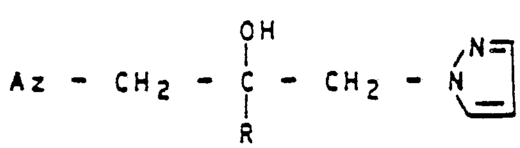

(I)

in which

Az represents 1,2,4-triazol-1-yl, imidazol-1-yl or pyrazol-1-yl and

R represents straight-chain or branched alkyl with 1 to 12 carbon atoms, straight-chain or branched alkenyl or alkinyl with in each case 2 to 6 carbon atoms, or phenyl, phenylalkyl with 1 to 4 carbon atoms in the alkyl part, phenylalkenyl with 2 to 4 carbon atoms in the alkenyl part or phenylalkinyl with 2 to 4 carbon atoms in the alkinyl part, each of which is optionally mono-, di- or tri-substituted in the phenyl part by identical or different substituents from the group comprising halogen, alkyl with 1 to 4 carbon atoms, alkoxy and alkylthio with in each case 1 or 2 carbon atoms, alkoxyalkyl with 1 or 2 carbon atoms in each alkyl part and phenyl which is optionally substituted by halogen ; or furthermore represents naphthyl, or cycloalkyl which has 3 to 7 carbon atoms and is optionally substituted by alkyl with 1 to 4 carbon atoms, and acid addition salts and metal salt complexes thereof.

2. Compounds of the formula (I) in Claim 1, wherein

Az represents 1,2,4-triazol-1-yl, imidazol-1-yl or pyrazol-1-yl and

R represents phenyl which is optionally monosubstituted or disubstituted by identical or different substituents from the group comprising fluorine, chlorine, methyl and methoxy.

3. Process for the preparation of 1-azolyl-3-pyrazolyl-2-propanol derivates of the general formula

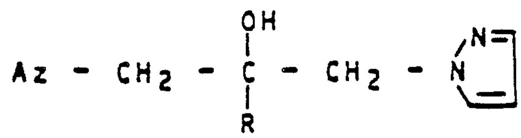

(I)

in which

Az represents 1,2,4-triazol-1-yl, imidazol-1-yl or pyrazol-1-yl and

R represents straight-chain or branched alkyl with 1 to 12 carbon atoms, straight-chain or branched alkenyl or alkinyl with in each case 2 to 6 carbon atoms, or phenyl, phenylalkyl with 1 to 4 carbon atoms in the alkyl part, phenylalkenyl with 2 to 4 carbon atoms in the alkenyl part or phenylalkinyl with 2 to 4 carbon atoms in the alkinyl part, each of which is optionally mono-, di- or tri-substituted in the phenyl part by identical or different substituents from the group comprising halogen, alkyl with 1 to 4 carbon atoms, alkoxy and alkylthio with in each case 1 or 2 carbon atoms, alkoxyalkyl with 1 or 2 carbon atoms in each alkyl part and phenyl which is optionally substituted by halogen ; or furthermore represents naphthyl, or cyloalkyl which has 3 to 7 carbon atoms and is optionally substituted by alkyl with 1 to 4 carbon atoms, and of the acid addition salts and metal salt complexes thereof,

characterised in that

a) 2-azolylmethyl-oxiranes of the formula

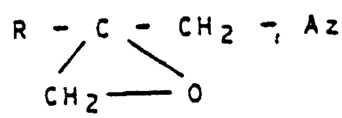

(II)

in which Az and R have the abovementioned meaning, are reacted with azoles of the formula

H—Az′ (III)

in which Az′ has the meanings of Az, but Az and/or Az′ represent pyrazol-1-yl, in the presence of an alkali metal alcoholate and in the presence of a diluent, or

b) 2-halogenomethyl-oxiranes of the formula

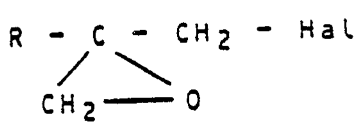

(IV)

in which

R has the abovementioned meaning and

Hal represents halogen,

are reacted with pyrazole of the formula

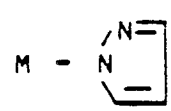

(V)

in which M represents hydrogen or an alkali metal, in the presence of a diluent and, if appropriate, in the presence of an acid-binding agent, or

c) dihalogenoalkanols of the formula

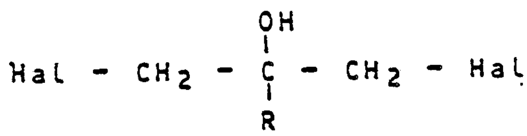

(VI)

in which Hal and R have the abovementioned meaning, are reacted with pyrazole of the formula (V) in the presence of a diluent and, if appropriate, in the presence of an acid-binding agent,
and, if appropriate, an acid or a metal salt is then added onto the compounds of the formula (I) thus obtained.

4. Fungicidal agents, characterised in that they contain at least one 1-azolyl-3-pyrazolyl-2-propanol derivative of the formula (I) according to Claim 1 or an acid addition salt or metal salt complex of a 1-azolyl-3-pyrazolyl-2-propanol derivative of the formula (I).

5. Method of combating fungi, characterised in that 1-azolyl-3-pyrazolyl-2-propanol derivatives of the formula (I) in Claim 1 or the acid addition salts or metal salt complexes thereof are allowed to act on fungi or their environment.

6. Use of 1-azolyl-3-pyrazolyl-2-propanol derivatives of the formula (I) in Claim 1 or of the acid addition salts or metal salt complexes thereof for combating fungi.

7. Process for the preparation of fungicidal agents, characterised in that 1-azolyl-3-pyrazolyl-2-propanol derivatives of the formula (I) in Claim 1 or the acid addition salts or metal salt complexes thereof are mixed with extenders and/or surface-active agents.

**Revendications**

1. Dérivés du 1-azolyl-3-pyrazolyl-2-propanol, de formule générale :

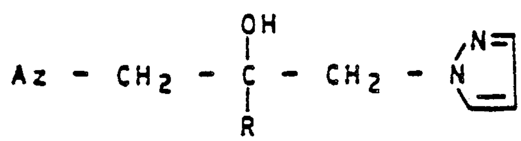

(I)

dans laquelle :

Az représente un groupe 1,2,4-triazole-1-yle, imidazole-1-yle ou pyrazole-1-yle, et

R représente un groupe alkyle linéaire ou ramifié ayant 1 à 12 atomes de carbone, un groupe alcényle et alcynyle, linéaire ou ramifié, ayant chacun 2 à 6 atomes de carbone, un groupe phényle substitué, phénylalkyle ayant 1 à 4 atomes de carbone dans la partie alkyle, phénylalcényle ayant 2 à 4 atomes de carbone dans la partie alcényle et phénylalcynyle ayant 2 à 4 atomes de carbone dans la partie alcynyle, la partie alkyle étant à chaque fois éventuellement substituée une à trois fois, de façon identique ou différente, par un reste halogène, alkyle ayant 1 à 4 atomes de carbone, alcoxy et alkylthio ayant chacun 1 ou 2 atomes de carbone, alcoxyalkyle ayant 1 ou 2 atomes de carbone dans chaque partie alkyle ainsi que par un reste phényle éventuellement substitué par de l'halogène ; en outre R représente un groupe naphtyle, ainsi qu'un groupe cycloalkyle ayant 3 à 7 atomes de carbone et qui est éventuellement substitué par un groupe alkyle ayant 1 à 4 atomes de carbone,
ainsi que leurs sels d'addition d'acides et leurs complexes avec des sels de métaux.

2. Composés de formule (I) de la revendication 1, dans lesquels :

Az représente un groupe 1,2,4-triazole-1-yle, imidazole-1-yle ou pyrazole-1-yle, et

R représente un groupe phényle éventuellement substitué une ou deux fois, de façon identique ou différente, par du fluor, du chlore, un groupe méthyle et un groupe méthoxy.

3. Procédé pour préparer des dérivés du 1-azolyl-3-pyrazolyl-2-propanol de formule générale :

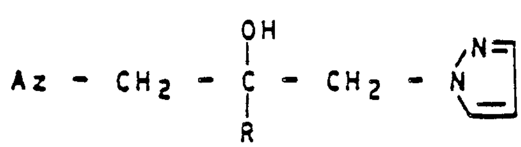

(I)

dans laquelle :

Az représente un groupe 1,2,4-triazole-1-yle, imidazole-1-yle ou pyrazole-1-yle, et

R représente un groupe alkyle linéaire ou ramifié ayant 1 à 12 atomes de carbone, un groupe alcényle et alcynyle linéaire ou ramifié ayant chacun 2 à 6 atomes de carbone, un groupe phényle substitué, phénylalkyle ayant 1 à 4 atomes de carbone dans la partie alkyle, phénylalcényle ayant 2 à 4 atomes de carbone dans la partie alcényle et phénylalcynyle ayant 2 à 4 atomes de carbone dans la partie alcynyle, chaque partie phényle étant éventuellement substituée, une à trois fois, de façon identique ou différente, par de l'halogène, par un reste alkyle ayant 1 à 4 atomes de carbone, alcoxy et alkylthio ayant chacun 1 ou 2 atomes de carbone, alcoxyalkyle ayant 1 ou 2 atomes de carbone dans chaque partie alkyle, ainsi que par un reste phényle éventuellement substitué par de l'halogène, R pouvant représenter en outre un groupe naphtyle ainsi qu'un groupe cycloalkyle ayant 3 à 7 atomes de carbone et qui est éventuellement substitué par un groupe alkyle ayant 1 à 4 atomes de carbone,
ainsi que de leurs sels d'addition d'acides et de leurs complexes de sels de métaux,
procédé caractérisé en ce que :

a) on fait réagir des 2-azolylméthyl-oxirannes de formule :

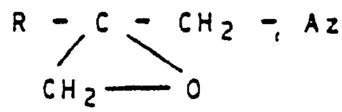

(II)

dans laquelle Az et R ont le sens indiqué ci-dessus, avec des azoles de formule :

$$H—Az' \quad (III)$$

dans laquelle : Az' a le sens de Az, Az et/ou Az' représentant cependant un groupe pyrazole-1-yle, en présence d'un alcoolate alcalin et en présence d'un diluant, ou

b) on fait réagir des 2-halogénométhyl-oxirannes de formule :

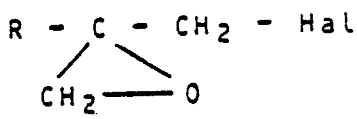

(IV)

dans laquelle :
R a le sens indiqué ci-dessus et
Hal représente un halogène,
avec un pyrazole de formule :

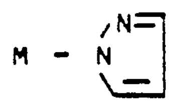

(V)

dans laquelle M représente un atome d'hydrogène ou un métal alcalin, en présence d'un diluant et éventuellement en présence d'un agent de fixation des acides, ou

c) on fait réagir des dihalogénoalcanols de formule :

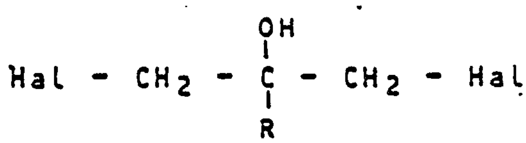

(VI)

dans laquelle Hal et R ont le sens indiqué ci-dessus, avec le pyrazole de formule (V), en présence d'un diluant et éventuellement en présence d'un agent de fixation des acides,
et éventuellement, sur les composés de formule (I) ainsi obtenus, on fixe ensuite par addition un acide ou un sel de métal.

4. Produit fongicide, caractérisé en ce qu'il contient au moins un dérivé de 1-azolyl-3-pyrazolyl-2-propanol de formule (I) selon la revendication 1, ou un sel d'addition d'acide ou un complexe de sel de métal d'un dérivé de 1-azolyl-3-pyrazolyl-2-propanol de formule (I).

5. Procédé pour combattre les champignons, caractérisé en ce qu'on laisse agir sur les champignons ou sur leur espace vital ou biotope des dérivés de 1-azolyl-3-pyrazolyl-2-propanol de formule (I) de la revendication 1, ou leurs sels d'addition d'acides ou leurs complexes avec des sels de métaux.

6. Utilisation des dérivés de 1-azolyl-3-pyrazolyl-2-propanol de formule (I) de la revendication 1, ou de leurs sels d'addition d'acides ou de leurs complexes avec des sels de métaux pour combattre des champignons.

7. Procédé pour préparer des produits fongicides, caractérisé en ce qu'on mélange des dérivés de 1-azolyl-3-pyrazolyl-2-propanol de formule (I) de la revendication 1, ou leurs sels d'addition d'acides ou leurs complexes avec des sels de métaux, avec des agents d'allongement et/ou avec des agents tensioactifs.